# EUROPEAN PATENT APPLICATION

(11) **EP 4 112 043 A1**
(43) Date of publication of application: **04.01.2023**
(21) Application number: 21382582.1
(22) Date of filing: 30.06.2021
(51) Int. Cl.: A61K 9/00, A61K 9/06, A61K 9/107, A61K 31/196, A61K 47/18, A61K 47/34, A61P 19/02, A61P 21/00, A61P 29/00, A61K 9/08

(54) **NANO-COMPLEX COMPOSITION COMPRISING DICLOFENAC**

(71) Applicant: GSK Consumer Healthcare SARL, 1197 Prangins (CH)
(72) Inventor: Alonso, Maria José, 15782 Santiago de Compostela (ES); Teijeiro, Desirée, 15782 Santiago de Compostela (ES); Ronsoni Zancán, Lali, 15782 Santiago de Compostela (ES)
(74) Representative: Haleon Patent Department

(57) **Abstract**

There is disclosed a nano-complex composition for topical drug delivery, comprising a diclofenac alkali metal salt, a cationic surfactant, diethylene glycol monoethyl ether, C₂ to C₃ alcohol, and water. The composition can further comprise hyaluronic acid. There is also provided a method of manufacture of nano-complex compositions for topical drug delivery. The compositions of the disclosure are useful in the treatment of joint pain, osteoarthritis, muscle pain, back pain and/or inflammation.

## Description

### FIELD OF THE INVENTION

This disclosure relates to formulations for the topical delivery of diclofenac. Specifically, it relates to novel nanocomplex formulations designed for about 2% to about 4% (w/w) diclofenac alkali metal salt. The formulations enable high and/or prolonged permeation of the active.

### BACKGROUND TO THE INVENTION

Topical, transdermal products comprising diclofenac, a non-steroidal anti-inflammatory drug (NSAID) of the acetic acid class, are currently available to patients and consumers in Europe and other countries, and are widely used for their analgesic and anti-inflammatory properties, providing an advantage over oral NSAID formulations, inter alia due to reduced systemic adverse effects. However, it is very difficult to develop topical formulations that are pharmaceutically acceptable and at the same time exhibit consumer preferred attributes. Additionally, diclofenac is a particularly challenging ingredient to work with, and poses many challenges to the formulator. Improved topical compositions comprising diclofenac are needed that meet pharmaceutical, technological and consumer expectations.

### SUMMARY OF THE INVENTION

There is provided a nano-complex composition for topical drug delivery, comprising:
a) a diclofenac alkali metal salt, in a concentration of about 2% to about 4% (w/w),
b) a cationic surfactant, in a concentration of about 0.5% to about 1.5% (w/w),
c) diethylene glycol monoethyl ether, in a concentration of about 1% to about 8% (w/w),
d) C₂ to C₃ alcohol, in a concentration of about 15% to about 25% (w/w), and
e) water.
In one embodiment, the nano-complex composition further comprises
f) hyaluronic acid, in a concentration of about 0.1% to about 2% (w/w).

In one embodiment, component d) is isopropanol in a concentration of about 15% to about 25% (w/w), ethanol in a concentration of about 15% to about 25% (w/w), or a combination of isopropanol and ethanol in a total concentration of about 15% to about 25% (w/w).

In one embodiment, component b) is a quaternary ammonium salt.

In one embodiment, component b) is a C₈-C₁₆-alkyl trimethylammonium salt.

In one embodiment, component e) is present in a concentration of about 65% to about 75% (w/w).

In one embodiment, the composition is self-assembling.

In one embodiment, the composition is self-assembling to an average nano-complex size of about 15nm to about 45nm, measured with dynamic light scattering technique.

In one embodiment, the composition is translucent.

In one embodiment, the composition has a zeta potential of about -12mV to about -4mV. In one embodiment, the composition has a pH of about 8 to about 8.5.

In one embodiment, the composition is for use in the treatment of joint pain, osteoarthritis, muscle pain, back pain and/or inflammation.

In one embodiment, the nano-complex composition is provided in a container, the container comprising an applicator for direct application onto the skin.

The disclosure also provides a method for manufacturing a nano-complex composition for topical drug delivery, the method comprising the steps of:
a) mixing water, and C₂ to C₃ alcohol;
b) adding to the mixture of a) a cationic surfactant and diethylene glycol monoethyl ether, and stirring until the resulting mixture is homogenic;
c) adding to the mixture of b) diclofenac alkali metal salt, and stirring until the diclofenac alkali metal salt is fully dissolved; and
d) adding to the mixture of c) water;
wherein, in the final composition, diclofenac alkali metal salt is present in a concentration of about 2% to about 4% (w/w), cationic surfactant is present in a concentration of about 0.5% to about 1.5% (w/w), diethylene glycol monoethyl ether is present in a concentration of about 1% to about 8% (w/w), and C₂ to C₃ alcohol is present in a concentration of about 15% to about 25% (w/w).

In one embodiment, hyaluronic acid is dissolved in water, and the solution comprising hyaluronic acid in water is used in step d), wherein hyaluronic acid is present in a concentration of about 0.1% (w/w) to about 2% (w/w) in the final composition.

### DETAILED DESCRIPTION OF THE INVENTION

To facilitate extended therapeutic efficacy, an important objective of the inventors has been to provide a composition which facilitates improved diclofenac penetration through the skin, into regions of interest for anti-inflammatory and anti-nociception effects. The skin, however, is a natural barrier, providing hydrophobic qualities to minimise percutaneous absorption of ionised drugs, such as diclofenac.

One way to improve penetration of diclofenac is through incorporation of higher concentrations of the active pharmaceutical ingredient (API). This however entails numerous difficulties. Higher amounts of API can lead to chemical instability due to a higher or lower pH. Higher amounts of API can also cause instability due to interaction with other ingredients of the formulation. The approach of raising API concentration is especially limited for diclofenac, due to the very limited solubility and high cristallisation tendency of diclofenac. Furthermore, higher API concentrations render products more expensive, and enlarge their environmental footprint. Inter alia for environmental reasons, it is desirable to achieve the highest possible penetration with the least concentration of active. Thereby, API usage could be reduced without compromising efficacy. Furthermore, exposure of the environment with "unused" active could be reduced.

Another way to achieve higher penetration of diclofenac is through incorporation of (higher) concentrations of permeation enhancer(s) in a formulation. However, permeation enhancer(s) can also cause skin irritation, and can, in some concentrations, even have a penetration retarding effect. Penetration enhancers can also lead to instability of multiphase topical systems. Additionally, product characteristics like rheology or appearance can be negatively impacted by inclusion of permeation enhancers. It would be preferred to provide a product that achieves high penetration of diclofenac even without the need to incorporate a permeation enhancer. But ideally, penetration enhancers could nevertheless be successfully incorporated into the formulation to further enhance penetration of the API, without a negative impact on stability, product attributes and safety. Thereby, a product range with different strengths or potencies could be created, based on the base formulation.

Several challenges lie in the manufacture of topical products. Many methods of manufacture are cumbersome, expensive and waste(water) intensive, e.g. because they require numerous vessels. Another problem specific to multiphase systems is the energy impact of usual emulsification methods. Using (high) shear homogenization, especially for prolonged periods of time, is energy intensive. This leads to higher production cost and can also negatively impact stability because of higher temperatures and oxygen introduction. Therefore, there is a need for a product that can be manufactured in an economic and environmentally friendly method of manufacture, without high energy impact.

An additional challenge is to provide a stable composition suitable for commercial distribution and sale. The minimum stability profile requires overcoming challenges including the resistance to phase separation in multiphase systems, creaming, precipitation and flocculation.

Another challenge is to provide a topical product with pleasant attributes. Currently, consumers prefer non greasy, light products. Also preferred are clear, transparent or translucent topical products. Ideally the products should have pleasing spread characteristics, and dry quickly without leaving residue on the skin. The texture of the product needs to be pleasant to consumers. Cooling properties are often preferred, however incorporation of excipients with a cooling effect is also limited due to various formulation challenges.

Consequently, there remains a need for a topical, transdermal diclofenac formulation that addresses one or more of the described challenges.

Unless indicated otherwise, concentrations are given in %(w/w).

The term "about" in relation to a numerical value x means, for example, x±10%, x±5%, x±4%, x±3%, x±2%, x±1%.

### PHARMACEUTICAL COMPOSITIONS

This disclosure provides pharmaceutical compositions for topical drug delivery which are nano-complex compositions with an aqueous outer phase. Nano-complexes are formed by combining oppositely charged (poly)electrolytes in suspension, which can spontaneously self-associate, primarily due to electrostatic forces. In the present disclosure, the oppositely charged electrolytes are diclofenac alkali metal salt (component a) in the disclosed compositions) and a cationic surfactant (component b) in the disclosed compositions). The nano-complexes are a so called inner phase, dispersed in the so called outer phase, also referred to as continuous phase, which is an aqueous phase. The nano-complexes comprise a "shell" of the oppositely charged electrolytes, and a core, the core comprising a solvent. Optionally, the nano-complexes further comprise hyaluronic acid. In other words, nano-complexes can be understood as nano-micelles with an "ionic shell", with the oppositely charged electrolytes as the micelle forming units. Charged, hydrophilic moieties, often called head groups, of components in the complex are oriented towards the outer aqueous phase, whilst uncharged, lipophilic moieties are directed towards the core of the complex. Nano-complexes are very well suited for the formulation of tailored pharmaceutical compositions for topical drug delivery. Additional ingredients can be incorporated within the core of the complex, in an area close to the charged head groups of the complex, at an interphase between the complex and the outer phase, or in the aqueous phase. Where an ingredient will accumulate depends on the exact composition, and the characteristics of the additional ingredient, such as hydrophilic lipophilic balance (HLB), partition coefficient and polarity. However, incorporating an additional ingredient, and even changing the concentration of an ingredient, can also destabilise the system, leading to flocculation, emulsion breaking, coalescence from nano-complexes to larger droplets, creaming or the like. Therefore, nano-complexes are a very delicate pharmaceutical form.

The present inventors have found pharmaceutical compositions for topical delivery of diclofenac, in the form of nano-complex compositions. In one embodiment, the compositions have a negative zeta potential of about -12mV to about -4 mV. In one embodiment, the nano-complexes have an average size (measured with dynamic light scattering technique) of about 15nm to about 45nm. In one embodiment, the nano-complexes have a narrow size variation which can be expressed in a polydispersion index (PDI) of about 0.1 to about 0.4.

The nano-complex compositions are self-assembling. In one embodiment, the nano-complex compositions are self nano-assembling. In one embodiment, the nano-complex compositions are self-assembling to an average nano-complex size of about 15nm to about 45nm. Thereby, the compositions can be manufactured with low-energy emulsification techniques.

The compositions disclosed herein comprise diclofenac alkali metal salt as the active pharmaceutical ingredient (API). In an embodiment, the diclofenac alkali metal salt is diclofenac sodium, diclofenac potassium, or a combination thereof. In a preferred embodiment, the composition comprises diclofenac sodium.

The nano-complex compositions preferably comprise diclofenac alkali metal salt in a concentration of about 2% to about 4% (w/w) (component **a**)). Preferably, the nano-complex compositions comprise about 2% to about 3.5% (w/w) diclofenac alkali metal salt. In one embodiment, the nano-complex compositions comprise about 2.15 (w/w) diclofenac alkali metal salt. In another embodiment, the nano-complex compositions comprise about 2.15 (w/w) diclofenac sodium salt. Diclofenac alkali metal salt, negatively charged in solution, contributes to the formation of the nano-complexes by providing the anionic charge.

The nano-complex compositions comprise a cationic surfactant (component **b**)). The cationic surfactant is important for the formation of the nano-complexes as discussed above. The cationic surfactant can have one positive charge per molecule or can have multiple positive charges per molecule. Preferably, the cationic surfactant has one positive charge per molecule.

The cationic surfactant is a cationic oil-in-water surfactant. In one embodiment, the cationic surfactant is a quaternary ammonium compound. Preferably, the cationic surfactant is an alkyl trimethylammonium or a dialkyl dimethylammonium salt or a combination thereof. Especially preferred are alkyl trimethylammonium salts. More preferred, the cationic surfactant is selected from the group of benzalkonium salt, benzethonium salt, cetylalkonium salt, cetylpyridinium salt, cetyltrimethylammonium salt, dequalinium salt, cetrimide and combinations thereof. In a preferred embodiment, the cationic surfactant is a C₈ to C₁₆-alkyl trimethylammonium salt. More preferred is C₁₁ to C₁₃ trimethylammonium salt. In a preferred embodiment, the cationic surfactant is cetrimide.

In one embodiment, the cationic surfactant is present in the compositions in a concentration of about 0.5% to about 1.5% (w/w). Preferably, the cationic surfactant is present in a concentration of about 1% (w/w).

The nano-complex compositions further comprise a solvent (component **c**)). The solvent can be a non-volatile solvent. Preferably, the solvent is a solvent of the glycol, or glycol ether or glycol ether ester family. Preferably, the solvent has a boiling point of above about 150°C at normal pressure. Preferably, the solvent has an octanol/water partition coefficient expressed as log P_{ow} of about -0.5 to about 1 at about 20°C. In one embodiment, the solvent is diethylene glycol monoethyl ether. In one embodiment, the solvent is diethylene glycol monoethyl ether.

In one embodiment, the solvent is present in a concentration of about 1% to about 8% (w/w). Preferably, the solvent is present in a concentration of about 2.5% to about 5% (w/w).

The nano-complex compositions further comprise C₂ to C₃ alcohol. In one embodiment, the C₂ to C₃ alcohol is selected from ethanol, isopropanol, and combinations thereof. In one embodiment, the nano-complex compositions comprise a combination of ethanol and isopropanol. In one embodiment, the ratio of ethanol to isopropanol is about 1:1 (w:w).

The C₂ to C₃ alcohol can be present in a concentration of about 10% to about 30% (w/w), preferably about 15% to about 25% (w/w). Preferably, the C₂ to C₃ alcohol is present in a concentration of about 20% (w/w). In one embodiment, only isopropanol is present in a concentration of about 15% to about 25% (w/w). In one embodiment, only ethanol is present in a concentration of about 15% to about 25% (w/w). In an alternative embodiment, the nano-complex compositions comprise a concentration of about 7% to about 12% (w/w) ethanol and about 7% to about 12% (w/w) isopropanol. These agents reduce the time that is needed to upon rubbing the composition into the skin until the skin feels dry (drying time). It was surprisingly found that said alcohols can successfully be incorporated into the nano-complex compositions, in the above concentration range.

As described above, the outer phase of the nano-complex compositions is water. It can be pharma grade water or purified water or any other water suitable for pharmaceutical compositions. In one embodiment, the nano-complex compositions comprise a concentration of at least about 60% (w/w) water. In another preferred embodiment, the nano-complex compositions comprise a concentration of about 65% to about 75% (w/w) water. Such a high water content is advantageous for topical compositions, because it can lead to a cooling skin sensation upon application, and because it lowers the total cost of the compositions. It is however challenging to formulate commercially acceptable compositions with a high water content, as these often take too long to dry on the skin, are not easy to rub in, and/or provide low solubilisation of the API.

Preferably, the nano-complex compositions are transparent or translucent. In one preferred embodiment, the nano-complex compositions are translucent. Translucent or transparent compositions are often preferred by patients and can lead to a better compliance. Compositions with an average complex size, determined by dynamic light scattering, of about 10nm to about 50nm, can be regarded as translucent. Nano-complex compositions with an average complex size, determined by dynamic light scattering, of about 5nm to about 10nm, can be regarded as transparent. Average complex size is the average size of the complex micelles present in the nano-complex composition, determined by dynamic light scattering.

Optionally, the nano-complex compositions can further comprise hyaluronic acid. Hyaluronic acid can be incorporated into the compositions as a shell-forming polymer. Preferred is high molecular weight hyaluronic acid. The nano-complexes that comprise a polymer can also be referred to as nano-capsules. The compositions that additionally comprise hyaluronic acid can thus also be referred to as nano-capsule compositions.

Hyaluronic acid can be present in a concentration of about 0.1% to about 2% (w/w). Preferred is a concentration of about 0.1 to about 0.5% (w/w). The concentration of hyaluronic acid can be increased up to about 2% (w/w), in a view to increase the viscosity of the compositions. The present inventors have found that hyaluronic acid can be incorporated in concentrations of up to about 2% (w/w) in the nano-capsule compositions of the present disclosure.

The nano-complex compositions can comprise further excipients, such as perfumes, chelating agents, preservatives, antioxidants or the like.

Preferably, the nano-complex compositions are self-assembling. Self-assembling compositions are two-phase systems that spontaneously form stable emulsions. This process involves complex interfacial hydrodynamic phenomena and depends on the system composition properties, and only very specific pharmaceutical excipient combinations lead to efficient self-emulsifying systems. Preferably, the nano-complex compositions spontaneously form nano-complexes with an average size (measured with dynamic light scattering technique) of the complexes of about 15nm to about 45nm. Self-assembling systems are preferred because they are more stable, less susceptible of phase separation, creaming flocculation and the like, and because they do not require high energy manufacturing methods like micro fluidization, high pressure homogenization or ultrasonic treatment or the like.

Preferably, the compositions have a pH suitable for topical administration. Preferably, the pH is in the range of about 8 to about 8.5. Preferably, the pH is inherent to the compositions without addition of an acid, base or buffer.

Preferably, the compositions are stable upon storage. Preferably, the compositions maintain homogenic without sign of phase separation when stored at about 25°C, about 60% relative humidity (RH), for about one month. More preferred, the compositions maintain homogenic without sign of phase separation when stored at about 25°C, about 60% RH, for about two months. More preferred, the compositions maintain homogenic without sign of phase separation when stored at about 25°C, about 60% RH, for about three months.

Preferably, the compositions maintain homogenic without sign of phase separation when stored at about 40°C, about 75% relative humidity (RH), for about one month. More preferred, the compositions maintain homogenic without sign of phase separation when stored at about 40°C, about 75% RH, for about two months. More preferred, the compositions maintain homogenic without sign of phase separation when stored at about 40°C, about 75% RH, for about three months.

Preferably, the compositions maintain a drug content of about 95% to about 105% of the initial drug content when stored at about 25°C, about 60% relative humidity (RH), for about one month. More preferred, the compositions maintain a drug content of about 95% to about 105% of the initial drug content when stored at about 25°C, about 60% RH, for about two months. More preferred, the compositions maintain a drug content of about 95% to about 105% of the initial drug content when stored at about 25°C, about 60% RH, for about three months. Preferably, the compositions maintain a drug content of about 95% to about 105% of the initial drug content when stored at about 40°C, about 75% relative humidity (RH), for about one month. More preferred, the compositions maintain a drug content of about 95% to about 105% of the initial drug content when stored at about 40°C, about 75% RH, for about two months. More preferred, the compositions maintain a drug content of about 95% to about 105% of the initial drug content when stored at about 40°C, about 75% RH, for about three months.

In one embodiment, the nano-complex composition comprises:
a) diclofenac alkali metal salt in a concentration of about 2% to about 4% (w/w),
b) a cationic surfactant in a concentration of about 0.5% to about 1.5% (w/w),
c) glycol, or glycol ether or glycol ether ester in a concentration of about 1% to about 8% (w/w),
d) C₂ to C₃ alcohol in a concentration of about 15% to about 25% (w/w), and
e) water.

In one embodiment, the nano-complex composition comprises:
a) diclofenac alkali metal salt in a concentration of about 2% to about 4% (w/w),
b) quaternary ammonium salt in a concentration of about 0.5% to about 1.5% (w/w),
c) diethylene glycol monoethyl ether in a concentration of about 1% to about 8% (w/w),
d) C₂ to C₃ alcohol in a concentration of about 15% to about 25% (w/w), and
e) water in a concentration of about 65% to about 75% (w/w).

In one embodiment, the nano-complex composition comprises:
a) diclofenac alkali metal salt in a concentration of about 2% to about 2.5% (w/w),
b) C₈-C₁₆-alkyl trimethylammonium salt in a concentration of about 0.5% to about 1.5% (w/w),
c) diethylene glycol monoethyl ether in a concentration of about 1% to about 8% (w/w),
d) ethanol or isopropanol or a mixture of ethanol and isopropanol in a concentration of about 15% to about 25% (w/w),
e) water in a concentration of about 65% to about 75% (w/w), and
f) hyaluronic acid, in a concentration of about 0.1% to about 2% (w/w).

In one embodiment, the nano-complex composition consists essentially of:
a) diclofenac sodium in a concentration of about 2% to about 2.5% (w/w),
b) cetrimide in a concentration of about 0.5% to about 1.5% (w/w),
c) diethylene glycol monoethyl ether in a concentration of about 5% (w/w),
d) mixture of ethanol and isopropanol in a ratio of about 1:1 (m:m) in a concentration of about 15% to about 25% (w/w), and
e) water q.s. in a concentration up to about 100% (w/w).

### METHODS OF MANUFACTURE

The compositions of this disclosure can be prepared as follows. A first aqueous solution is prepared from 70 volume% of the water of a given formulation and the C₂ to C₃ alcohol, the solvent such as diethylene glycol monoethyl ether and the diclofenac alkali metal salt. The solution is stirred at about 40°C for about 30 minutes, or until the diclofenac salt is completely dissolved, e.g. at about 700 rotations per minute (rpm) with a magnetic stirrer. The cationic surfactant is weighed and added to the solution and stirring is continued at the same temperature and speed for about another 30 minutes, or until the cationic surfactant is completely dissolved. For compositions comprising hyaluronic acid, a second aqueous solution is prepared by dissolving the hyaluronic acid in the remainder of the water under stirring. If no hyaluronic acid is used, the second aqueous solution is only the remainder of the water. The two solutions are mixed under vigorous magnetic stirring (about 700 rotations per minute, rpm), for about 30 minutes at room temperature.

For big scale batches, the first aqueous solution can be mixed at about 300 rpm and about 40°C for about 30 minutes using an overhead propeller stirrer, to obtain a clear and homogenic solution. After that, the second aqueous solution (with or without hyaluronic acid) is added at a flow rate of about 150 mL/min under stirring at about 400 rpm at room temperature. After the complete addition of the aqueous phase, the stirring can be increased to about 700 rpm for about 10 minutes, and then reduced again to about 300 rpm for about another 20 minutes.

### METHODS OF TREATMENT

The nano-complex composition can be administered to mammals suffering from joint pain, osteoarthritis, muscle pain, back pain and/or inflammation. Preferably, the nano-complex compositions are for the treatment of humans. The nano-complex composition are useful in the treatment of joint pain, osteoarthritis, muscle pain, back pain and/or inflammation. The methods of treatment can comprise administering to a subject in need thereof a pharmaceutically effective amount of the micellar solution or gel compositions disclosed herein. The methods can comprise administering the composition to skin that covers a body part suffering from or causing one or more of joint pain, osteoarthritis, muscle pain, back pain and/or inflammation.. They can be rubbed in until the skin feels dry. They can be applied 2-4 times per day. Alternatively, they may be applied once per day. Typical doses of the micellar solutions comprise 0.09mg diclofenac equivalent/ cm² (2-4 times per day application) or 0.21mg diclofenac equivalent/ cm² (twice per day application).

The nano-complex composition can be provided in a container comprising an applicator for direct application onto the skin. The nano-complex composition can be provided in a package comprising a means for dosing. In one embodiment, the dosing means is a pump. In one embodiment, the means for dosing is a pipette.

### EXAMPLES

### Initial screening

In a formulation screening, 24 formulations were developed using different ratios of excipients and diclofenac listed in Table 1.

**Table 1: Summary of composition for formulation screening.**

| Component | Concentration [% (w/w)] |
|---|---|
| Cetrimide | 1.0 |
| Hyaluronic Acid | 0.0 to 0.3 |
| Ethanol | 20 |
| Transcutol P | 1.0 to 8.0 |
| Diclofenac sodium | 2.5 to 3.5 |
| Water | 71 to 79 |

Formulations were prepared by solubilisation of diclofenac sodium in a hydroalcoholic solution containing a portion of the water, ethanol, diethylene glycol monoethyl ether and cetrimide, and subsequent addition of the rest of the water (with/without hyaluronic acid) over the former solution under continuous stirring. The formulations were screened for visual appearance (transparent or translucent), self-assembly and initial stability (no immediate phase separation/ flocculation/ cristallisation).

Out of the 24 formulations, ten formulations with the best visual characteristics were selected for characterization and ICH stability studies. Their composition is reported in Table 2.

**Table 2: Quantitative composition of selected nanocomplex formulations.**

| Formulation code name | Concentration [%(w/w)] | | | | |
|---|---|---|---|---|---|
| | Hyaluronic acid | Ethanol | Transcutol P | Cetrimide | DiclofenacNa |
| N9 | 0.3 | 20 | 1 | 1 | 2.15 |
| N10 | 0.3 | 20 | 5 | 1 | 2.15 |
| N11 | 0.3 | 20 | 8 | 1 | 2.15 |
| N12 | 0 | 20 | 1 | 1 | 2.15 |
| N13 | 0 | 20 | 5 | 1 | 2.15 |
| N14 | 0 | 20 | 8 | 1 | 2.15 |
| N15 | 0.3 | 20 | 2.5 | 1 | 2.15 |
| N16 | 0.3 | 20 | 5 | 1 | 3.5 |
| N17 | 0 | 20 | 2.5 | 1 | 2.15 |
| N18 | 0 | 20 | 5 | 1 | 3.5 |

Physico-chemical characteristics of the ten formulations of Table 2 are reported in Table 3.

Nine out of the ten formulations (coded as N9, N10, N11, N12, N13, N15, N16, N17 and N18), in addition to the initial visual characteristics, showed advantageous characteristics in terms of complex size, PDI, visual homogeneity, and drug content.

**Table 3: Physico-chemical characteristics of selected nanocomplex formulations.**

| Formulation code name | Complex size [nm] | Zeta potential [mV] | pH | Drug content (% vs. initial) |
|---|---|---|---|---|
| N9 | 36 | -10 | 8.3 | 105 |
| N10 | 103 | -8 | 8.2 | 101 |
| N11 | 153 | -6 | 8.2 | Not determined |
| N12 | 30 | -17 | 8.6 | 97 |
| N13 | 29 | -6 | 8.2 | 95 |
| N14 | 36 | -3 | 8.3 | Not Determined |
| N15 | 32 | -10 | 8.4 | 103 |
| N16 | 378 | -8 | 8.4 | 103 |
| N17 | 30 | -7 | 8.2 | 98 |
| N18 | 45 | -3 | 8.5 | 104 |

Embodiments described herein can be understood more readily by reference to the detailed description and examples. Elements and methods described herein, however, are not limited to the specific embodiments presented in the detailed description, and examples. It should be recognized that the exemplary embodiments herein are merely illustrative of the principles of the present invention. Numerous modifications and adaptations will be readily apparent to those of skill in the art without departing from the spirit and scope of the invention.

In addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "5 to 10" or "5-10" should generally be considered to include the end points 5 and 10.

It is further to be understood that the feature or features of one embodiment may generally be applied to other embodiments, even though not specifically described or illustrated in such other embodiments, unless expressly prohibited by this disclosure or the nature of the relevant embodiments. Likewise, compositions and methods described herein can include any combination of features and/or steps described herein not inconsistent with the objectives of the present disclosure. Numerous modifications and/or adaptations of the compositions and methods described herein will be readily apparent to those skilled in the art without departing from the present subject matter.

## Claims

1. A nano-complex composition for topical drug delivery, comprising:
a) a diclofenac alkali metal salt, in a concentration of about 2% to about 4% (w/w),
b) a cationic surfactant, in a concentration of about 0.5% to about 1.5% (w/w),
c) diethylene glycol monoethyl ether, in a concentration of about 1% to about 8% (w/w),
d) C₂ to C₃ alcohol, in a concentration of about 15% to about 25% (w/w), and
e) water.

2. A nano-complex composition according to claim 1, further comprising:
f) hyaluronic acid, in a concentration of about 0.1% to about 2% (w/w).

3. A nano-complex composition according to claim 1 or 2, wherein component d) is isopropanol, ethanol, or a combination of isopropanol and ethanol.

4. A composition according to claim 1, 2 or 3, wherein component b) is a quaternary ammonium salt.

5. A composition according to any of the preceding claims, wherein component b) is a C₈-C₁₆-alkyl trimethylammonium salt.

6. A composition according to any of the preceding claims, wherein component e) is present in a concentration of about 65% to about 75% (w/w).

7. A composition according to any of the preceding claims, wherein the composition is self-assembling.

8. A composition according to claim 7, wherein the composition is self-assembling to an average nano-complex size of about 15nm to about 45nm, measured with dynamic light scattering technique.

9. A composition according to any of the preceding claims, wherein the composition is translucent.

10. A composition according to any of the preceding claims, wherein the composition has a zeta potential of about -12mV to about -4mV.

11. A composition according to any of the preceding claims, wherein the composition has a pH of about 8 to about 8.5.

12. A composition according to any of the preceding claims, for use in the treatment of joint pain, osteoarthritis, muscle pain, back pain and/or inflammation.

13. A composition according to any of the preceding claims, provided in a container, the container comprising an applicator for direct application onto the skin.

14. A method for manufacturing a nano-complex composition for topical drug delivery, the method comprising the steps of:
**a)** mixing water, and C₂ to C₃ alcohol;
**b)** adding to the mixture of a) a cationic surfactant, and diethylene glycol monoethyl ether, and stirring until the resulting mixture is homogenic;
**c)** adding to the mixture of b) diclofenac alkali metal salt, and stirring until the diclofenac alkali metal salt is fully dissolved; and
**d)** adding to the mixture of c) water;
wherein, in the final composition, diclofenac alkali metal salt is present in a concentration of about 2% to about 4% (w/w), cationic surfactant is present in a concentration of about 0.5% to about 1.5% (w/w), diethylene glycol monoethyl ether is present in a concentration of about 1% to about 8% (w/w), and C₂ to C₃ alcohol is present in a concentration of about 15% to about 25% (w/w).

15. A method according to claim 14, wherein hyaluronic acid is dissolved in water, and the solution comprising hyaluronic acid in water is used in step d), wherein hyaluronic acid is present in a concentration of about 0.1% (w/w) to about 2% (w/w) in the final composition.
